(19) Europäisches Patentamt
European Patent Office
Office européen des brevets

(11) **EP 3 666 176 A1**

(12) **EUROPEAN PATENT APPLICATION**

(43) Date of publication:
**17.06.2020 Bulletin 2020/25**

(51) Int Cl.:
**A61B 5/00** (2006.01)

(21) Application number: **18212799.3**

(22) Date of filing: **14.12.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
- **Bourquin, Yannyk Parulian Julian**
  **5656 AE Eindhoven (NL)**
- **Shan, Caifeng**
  **5656 AE Eindhoven (NL)**
- **Lai, Marco**
  **5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **APPARATUS FOR DETECTING TISSUE INFLAMMATION**

(57) There is provided an apparatus (100) for detecting tissue inflammation. The apparatus (100) comprises a processor (102) configured to acquire, from at least one sensor (104), a plurality of photoplethysmography, PPG, signals indicative of light detected in a region of tissue at a plurality of respective locations within the region. The processor is also configured to process the acquired plurality of PPG signals to determine an amplitude and a phase of each of the plurality of PPG signals and detect tissue inflammation based on the determined amplitude and phase of each of the plurality of PPG signals.

Fig. 4

**Description**

FIELD OF THE INVENTION

The disclosure relates to an apparatus and a method for detecting tissue inflammation.

BACKGROUND OF THE INVENTION

Tissue inflammation can provide a useful insight into the appearance and health of tissue. It is part of the complex biological response of body tissues to harmful stimuli, such as pathogens, damaged cells, or irritants, and is a protective response involving immune cells, blood vessels, and molecular mediators.

An example of tissue inflammation is a pimple. A pimple is a small inflamed elevation of the skin. It is a result of excess oil becoming trapped in the pores of the skin, which causes inflammation and infection. Pimples occur at the location of sebaceous ducts, which transport sebum from the sebaceous glands to the skin. Driven by hormonal changes at the onset of puberty, the sebaceous glands start to produce more sebum. As this oily substance is a rich food source for skin bacteria like propionibacterium acnes, the sebaceous duct becomes populated by these bacteria. Their consumptive presence results in a changing sebum composition characterized by a higher concentration of lipid peroxides and free fatty acids. These somewhat aggressive agents and the propionibacterium acnes bacteria themselves present the trigger for pre-inflammatory processes in the perifollicular area.

Generally, children do not have pimples before the age of 12. However, after the onset of puberty, over 85% of teenagers develop pimples with frequent breakouts that generally continue until the age of 25 or so. In fact, even beyond this age people can still have pimples. Pimples are a clinical manifestation of acne, which is a skin condition that affects many people. Acne consists of papules and pustules, which are both inflamed elevated lesions and only differ in the sense that pustules contain a visible amount of pus, while papules do not. The early detection of pimples to determine the onset acne is useful.

In fact, the early detection of tissue inflammation generally can be useful. A tissue inflammation is characterized by local increased (or enhanced) perfused areas. An early stage pimple starts with inflammation (which causes increased perfusion), even before the pimple becomes noticeable to the user. A local enhanced perfused area can be detected by laser scattering technologies, such as laser Doppler perfusion imaging or laser speckle contrast analysis. WO 2007/103795 discloses using spectral imaging technology to compare an erythema map of imaged skin to a visible-light image of the imaged skin to identify any area of per-emergent lesion.

However, the existing technologies for detecting tissue inflammation are costly, with limited motion robustness, and are hard to commercialize with off-the-shelf components. Also, in the existing techniques, non-inflammatory tissue may be misclassified as inflammatory tissue where the tissue is marked, e.g. comprises a tattoo, make-up, spider vein, etc. As such, the existing techniques lack accuracy in detecting inflammatory tissue.

SUMMARY OF THE INVENTION

As noted above, a limitation with existing techniques is that non-inflammatory tissue may be misclassified as inflammatory tissue and thus the existing techniques lack accuracy in detecting inflammatory tissue. It would thus be valuable to have an improvement to address this limitation.

Therefore, according to a first aspect, there is provided an apparatus for detecting tissue inflammation. The apparatus comprises a processor configured to acquire, from at least one sensor, a plurality of photoplethysmography (PPG) signals indicative of light detected in a region of tissue at a plurality of respective locations within the region. The processor is also configured to process the acquired plurality of PPG signals to determine an amplitude and a phase of each of the plurality of PPG signals and detect tissue inflammation based on the determined amplitude and phase of each of the plurality of PPG signals.

In some embodiments, the processor may be configured to detect tissue inflammation if the determined amplitude of at least one of the plurality of PPG signals is greater than a threshold amplitude. In some embodiments, the amplitude of each of the plurality of PPG signals may be the amplitude of the PPG signal normalized by a direct current component of the PPG signal.

In some embodiments, the processor may be configured to detect tissue inflammation if a phase shift is detected in the determined phase of at least one of the plurality of PPG signals. In some embodiments, the processor may be configured to detect tissue inflammation if the detected phase shift is greater than a threshold phase shift. In some embodiments, the threshold phase shift may be a phase shift in a range from 45 degrees to 135 degrees.

In some embodiments, the processor may be configured to generate an amplitude map representative of the determined amplitude of each of the plurality of PPG signals. In some embodiments, the processor may be configured to generate an amplitude map representative of the determined amplitude of each of the plurality of PPG signals for which the determined amplitude is greater than a threshold amplitude.

In some embodiments, the processor may be configured to generate a phase map representative of the determined phase of each of the plurality of PPG signals. In some embodiments, the processor may be configured to generate a phase map representative of the determined phase of each of the plurality of PPG signals for which a phase shift detected in the determined phase is greater than a threshold phase shift.

In some embodiments, the processor may be configured to compare the generated amplitude map and the generated phase map and determine a location of the detected tissue inflammation in the region of tissue based on the comparison.

In some embodiments, the processor may be configured to generate a tissue inflammation map representative of the determined location of the detected tissue inflammation in the region of tissue. In some embodiments, the processor may be configured to acquire an image of the region of tissue and indicate the determined location of the detected tissue inflammation from the generated tissue inflammation map on the acquired image of the region of tissue.

According to a second aspect, there is provided a method for detecting tissue inflammation. The method comprises acquiring a plurality of PPG signals indicative of light detected in a region of tissue at a plurality of respective locations within the region and processing the acquired plurality of PPG signals to determine an amplitude and a phase of each of the plurality of PPG signals. The method also comprises detecting tissue inflammation based on the determined amplitude and phase of each of the plurality of PPG signals.

According to a third aspect, there is provided a computer program product comprising a computer readable medium. The computer readable medium has computer readable code embodied therein. The computer readable code is configured such that, on execution by a suitable computer or processor, the computer or processor is caused to perform the method described earlier.

According to the aspects and embodiments described above, the limitations of existing techniques are addressed. In particular, according to the above-described aspects and embodiments, both the amplitude and the phase of each of the plurality of PPG signals is used in the detection of tissue inflammation. This prevents the misclassification of non-inflammatory tissue (such as where the tissue is marked, e.g. comprises a tattoo, make-up, spider vein, etc.) as inflammatory tissue and thus inflammatory tissue can be detected more accurately. There is thus described an improved apparatus and method for detecting tissue inflammation.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1 is a schematic illustration of an apparatus according to an embodiment;
Fig. 2 is a flow chart illustrating a method according to an embodiment;
Fig. 3 is a block diagram illustrating a method according to an embodiment; and
Figs. 4-8 are each illustrations of amplitude and phase maps.

DETAILED DESCRIPTION OF THE EMBODIMENTS

As noted above, there is provided herein an improved apparatus and method for detecting tissue inflammation. In some embodiments, the apparatus described herein may be a dedicated apparatus (or device) for detecting tissue inflammation or a mobile device such as a smartphone or tablet (e.g. running an application or "app" for detecting tissue inflammation).

The tissue referred to herein may comprise any type of tissue. For example, the tissue may comprise epithelial tissue such as skin (e.g. the epidermis of the skin, the cells of the skin, hair follicles in the skin, or similar) or the lining of hollow organs (e.g. the lining of gastrointestinal tract organs or any other hollow organs), muscle tissue (e.g. cardiac muscle tissue, skeletal muscle, or any other muscle tissue), connective tissue (e.g. fat, bone, tendons, or any other connective tissue), nervous tissue (e.g. the brain, spinal cord, nerves, or any other nervous tissue), blood vessels, or any other tissue, or any combination of tissue.

Generally, tissue inflammation is an (e.g. localized) inflammation of tissue. Tissue inflammation can be the biological response of the tissue to harmful stimuli, such as pathogens, damaged cells, irritants, etc. Tissue inflammation can cause the tissue to become reddened, swollen and/or hot. There are various causes of tissue inflammation and examples include, but are not limited to, a pustule (or pimple), a papule, a cyst, a nodule, a cut, etc.

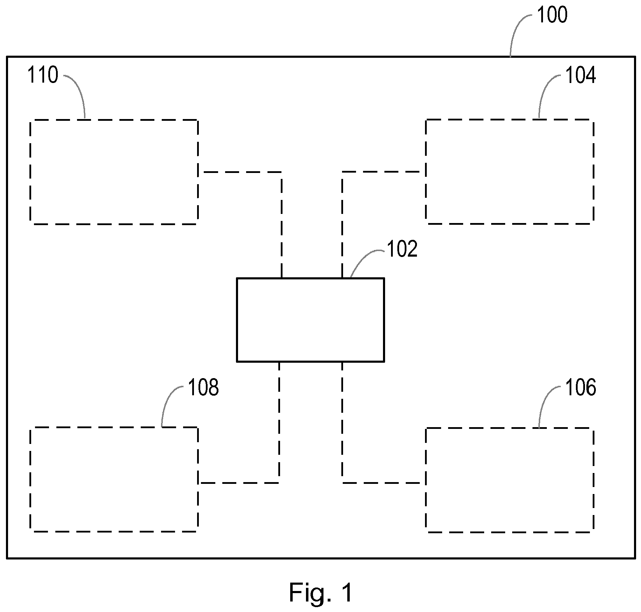

Fig. 1 illustrates an apparatus 100 for detecting tissue inflammation according to an embodiment. As illustrated in Fig. 1, the apparatus 100 comprises a processor 102. Briefly, the processor 102 is configured to acquire a plurality of photoplethysmography (PPG) signals indicative of light detected in a region of tissue at a plurality of respective locations within the region. The region of tissue referred to herein may also be referred to as a region of interest (ROI) of the tissue. The processor 102 is configured to acquire the plurality of PPG signals from at least one sensor 104. The processor 102 is also configured to process the acquired plurality of PPG signals to determine an amplitude and a phase of each of the plurality of PPG signals. The processor 102 is configured to detect tissue inflammation based on the determined amplitude and phase of each of the plurality of PPG signals.

The processor 102 can be implemented in numerous ways, with software and/or hardware, to perform the various functions described herein. In particular implementations, the processor 102 can comprise a plurality of software and/or hardware modules, each configured to perform, or that are for performing, individual or multiple steps of the method described herein. The processor 102 may comprise one or more processors (such as one or more microprocessors, one or more multi-core processors and/or one or more digital signal processors (DSPs)), one or more processing units, and/or one or more controllers (such as one or more microcontrollers) that may be configured or programmed (e.g. using software or computer program code) to perform the various functions described herein. The processor 102 may be implemented as a combination of dedicated hardware (e.g. amplifiers, pre-amplifiers, analog-to-digital convertors (ADCs) and/or digital-to-analog convertors (DACs)) to perform some functions and a processor (e.g. one or more programmed microprocessors, DSPs and associated circuitry) to perform other functions.

As illustrated in Fig. 1, in some embodiments, the apparatus 100 can comprise at least one sensor 104 from which the processor 102 can be configured to acquire one or more of the plurality of PPG signals. Alternatively or in addition, at least one sensor 104 from which the processor 102 can be configured to acquire one or more of the plurality of PPG signals may be external to (e.g. separate to or remote from) the apparatus 100. The at least one sensor 104 referred to herein is configured to obtain the plurality of PPG signals from the region of tissue at the plurality of respective locations within the region. Thus, the at least one sensor 104 may also be referred to as at least one PPG sensor. The at least one sensor 104 may be configured to obtain the plurality of PPG signals at a single (e.g. a certain) wavelength of light or at multiple wavelengths of light. The at least one sensor 104 may be a single sensor or a plurality (e.g. a series) of sensors.

Examples of the at least one sensor 104 include, but are not limited to, a pulse oximeter (which can be configured to illuminate the region of tissue and measure changes in light absorption within the region of tissue to acquire the plurality of PPG signals), a camera (which can be configured to measure changes in tissue color to acquire the plurality of PPG signal), or any other sensor or combination of sensors suitable for obtaining PPG signals. A person skilled in the art will be aware of a variety of sensors suitable for acquiring the plurality of PPG signals and the manner in which those sensors may be configured to operate to acquire the plurality of PPG signals. In some embodiments, at least one sensor 104 may be positioned at distance from (e.g. remote from) the tissue. Thus, the at least one sensor 104 can be a non-contact sensor according to some embodiments. The plurality of PPG signals acquired in these embodiments may thus also be referred to as a plurality of non-contact PPG signals. Alternatively or in addition, in some embodiments, at least one sensor 104 may be positioned such that it contacts the tissue to obtain the plurality of PPG signals.

As illustrated in Fig. 1, in some embodiments, the apparatus 100 may comprise at least one memory 106. Alternatively or in addition, at least one memory 106 may be external to (e.g. separate to or remote from) the apparatus 100. For example, a hospital database may comprise at least one memory 106, at least one memory 106 may be a cloud computing resource, or similar. The processor 102 of the apparatus 100 may be configured to communicate with and/or connect to at least one memory 106. A memory 106 may comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM), and electrically erasable PROM (EEPROM). In some embodiments, at least one memory 106 can be configured to store program code that can be executed by the processor 102 of the apparatus 100 to cause the apparatus 100 to operate in the manner described herein.

Alternatively or in addition, in some embodiments, at least one memory 106 can be configured to store information required by or resulting from the method described herein. For example, in some embodiments, at least one memory 106 may be configured to store any one or more of the acquired plurality of PPG signals, the determined amplitude of one or more of the plurality of PPG signals, the determined phase of one or more of the plurality of PPG signals, the detected tissue inflammation, or any other information, or any combination of information, required by or resulting from the method described herein. In some embodiments, the processor 102 of the apparatus 100 can be configured to control at least one memory 106 to store information required by or resulting from the method described herein.

As illustrated in Fig. 1, in some embodiments, the apparatus 100 may comprise a user interface 108. Alternatively or in addition, the user interface 108 may be external to (e.g. separate to or remote from) the apparatus 100. The processor 102 of the apparatus 100 may be configured to communicate with and/or connect to a user interface 108. In some embodiments, the processor 102 of the apparatus 100 can be configured to control the user interface 108 to operate in the manner described herein.

The user interface 108 can be configured to render (or output, display, or provide) information required by or resulting from the method described herein. For example, in some embodiments, the user interface 108 may be configured to render (or output, display, or provide) one or more of the acquired plurality of PPG signals, the determined amplitude of one or more of the plurality of PPG signals, the determined phase of one or more of the plurality of PPG signals, the detected tissue inflammation, or any other information, or any combination of information, required by or resulting from the method described herein. Alternatively or in addition, the user interface 108 can be configured to receive a user input. For example, the user interface 108 may allow a user to manually enter information or instructions, interact with and/or control the apparatus 100. Thus, the user interface 108 may be any user interface that enables the rendering (or outputting, displaying, or providing) of information and, alternatively or in addition, enables a user to provide a user input.

For example, the user interface 108 may comprise one or more switches, one or more buttons, a keypad, a keyboard, a mouse, a touch screen or an application (for example, on a smart device such as a tablet, a smartphone, or any other smart device), a display or display screen, a graphical user interface (GUI) such as a touch screen, or any other visual component, one or more speakers, one or more microphones or any other audio component, one or more lights (such as light emitting diode LED lights), a component for providing tactile or haptic feedback (such as a vibration function, or any other tactile feedback component), an augmented reality device (such as augmented reality glasses, or any other augmented reality device), a smart device (such as a smart mirror, a tablet, a smart phone, a smart watch, or any other smart device), or any other user interface, or combination of user interfaces. In some embodiments, the user interface that is controlled to render information may be the same user interface as that which enables the user to provide a user input.

As illustrated in Fig. 1, in some embodiments, the apparatus 100 may comprise a communications interface (or communications circuitry) 110. Alternatively or in addition, the communications interface 110 may be external to (e.g. separate to or remote from) the apparatus 100. The communications interface 110 can be for enabling the apparatus 100, or components of the apparatus 100, to communicate with and/or connect to one or more other components (e.g. one or more sensors, interfaces, devices, processors, or memories), such as any of those described herein. For example, the communications interface 110 can be for enabling the processor 102 of the apparatus 100 to communicate with and/or connect to any one or more of the at least one sensor 104 mentioned earlier, the at least one memory 106 mentioned earlier, and the user interface 108 mentioned earlier.

The communications interface 110 may enable the apparatus 100, or components of the apparatus 100, to communicate and/or connect in any suitable way. For example, the communications interface 110 may enable the apparatus 100, or components of the apparatus 100, to communicate and/or connect wirelessly, via a wired connection, or via any other communication (or data transfer) mechanism. In some wireless embodiments, for example, the communications interface 110 may enable the apparatus 100, or components of the apparatus 100, to use radio frequency (RF), Bluetooth, or any other wireless communication technology to communicate and/or connect.

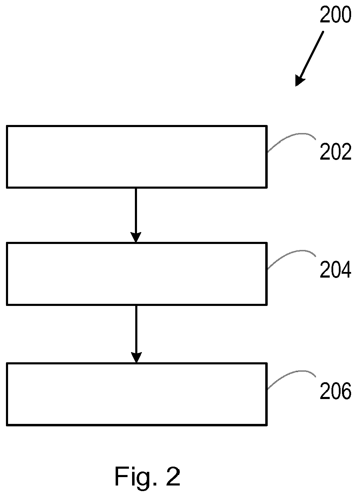

Fig. 2 illustrates a method 200 for detecting tissue inflammation according to an embodiment. More specifically, Fig. 2 illustrates a method 200 of operating the apparatus 100 described earlier for detecting tissue inflammation. The method 200 is a computer-implemented method. As described earlier, the apparatus 100 comprises a processor 102. The method 200 illustrated in Fig. 2 can generally be performed by or under the control of the processor 102 of the apparatus 100.

With reference to Fig. 2, at block 202 of Fig. 2, a plurality of PPG signals indicative of light detected in a region of tissue at a plurality of respective locations within the region are acquired. More specifically, the processor 102 of the apparatus 100 acquires the plurality of PPG signals from at least one sensor 104.

The light detected in the region of tissue can be indicative of the color in the region of tissue, e.g. changes in the light detected in the region of tissue can be indicative of changes in the color in the region of tissue. The changes in color in the region of tissue are caused by the pulse pressure. Thus, the plurality of PPG signals may also be referred to as a plurality of pulse signals. Generally, each PPG signal comprises a direct current (DC) component and an alternating current (AC) component. The DC component of each PPG signal can be attributable to the bulk absorption of the tissue in the respective location within the region of tissue. The AC component of each PPG signal can be attributable to the variation in blood volume in the respective location within the region of tissue, which is caused by the pulse pressure.

Returning back to Fig. 2, at block 204, the acquired plurality of PPG signals are processed to determine an amplitude and a phase of each of the plurality of PPG signals. More specifically, the processor 102 of the apparatus 100 processes the acquired plurality of PPG signals to determine an amplitude and a phase of each of the plurality of PPG signals. As the plurality of PPG signals are indicative of light detected in the region of tissue at a plurality of respective locations within the region, the amplitude and phase of each of the plurality of PPG signals is effectively determined at the plurality of respective locations within the region.

At block 206 of Fig. 2, tissue inflammation is detected based on the determined amplitude and phase of each of the plurality of PPG signals. More specifically, the processor 102 of the apparatus 100 detects tissue inflammation based on the determined amplitude and phase of each of the plurality of PPG signals. Thus, the detection of tissue inflammation is made by combining amplitude and phase information.

In some embodiments, the processor 102 may be configured to detect tissue inflammation if the determined amplitude of at least one of the plurality of PPG signals is greater than a threshold amplitude. The threshold amplitude may be set differently depending on the subject and/or conditions. Thus, in some embodiments, the threshold amplitude may be determined through calibration where tests are performed on various subjects and/or under various conditions in order to determine an optimum threshold amplitude.

In some embodiments, the amplitude of each of the plurality of PPG signals may be a normalized amplitude. For example, the amplitude of each of the plurality of PPG signals may be the amplitude of the (raw) PPG signal normalized by the direct current (DC) component of the PPG signal. In some of these embodiments, the threshold amplitude may be an amplitude in a range from 0.2 to 2, for example an amplitude in a range from 0.3 to 1.9, for example an amplitude in a range from 0.4 to 1.8, for example an amplitude in a range from 0.5 to 1.7, for example an amplitude in a range from 0.6 to 1.6, for example an amplitude in a range from 0.7 to 1.5, for example an amplitude in a range from 0.8 to 1.4, for example an amplitude in a range from 0.9 to 1.3, for example an amplitude in a range from 1.0 to 1.2. In other embodiments, the processor 102 may be configured to generate an amplitude map histogram representative of the determined amplitude of each of the plurality of PPG signals and the threshold amplitude may be an amplitude in a range from 40 to 100% of the amplitude map histogram.

Alternatively or in addition, in some embodiments, the processor 102 may be configured to detect tissue inflammation if a phase shift is detected in the determined phase of at least one of the plurality of PPG signals. Thus, the processor 102 may be configured to detect tissue inflammation if the determined amplitude of at least one of the plurality of PPG signals is greater than a threshold amplitude, or the processor 102 may be configured to detect tissue inflammation if a phase shift is detected in the determined phase of at least one of the plurality of PPG signals, or the processor 102 may be configured to detect tissue inflammation if the determined amplitude of at least one of the plurality of PPG signals is greater than a threshold amplitude and a phase shift is detected in the determined phase of at least one of the plurality of PPG signals. In some embodiments, this latter detection can be achieved using a logic gate (e.g. the "AND" logic gate) or a more complex algorithm (e.g. optimal weighting factors may be learned from a collected data set about tissue inflammation detection).

In some embodiments where a phase shift is detected, the processor 102 may be configured to detect tissue inflammation if the detected phase shift is greater than a threshold phase shift. In some embodiments, the threshold phase shift may be a phase shift in a range from 45 to 135 degrees, for example a phase shift in a range from 50 to 130 degrees, for example a phase shift in a range from 55 to 125 degrees, for example a phase shift in a range from 60 to 120 degrees, for example a phase shift in a range from 65 to 115 degrees, for example a phase shift in a range from 70 to 110 degrees, for example a phase shift in a range from 75 to 105 degrees, for example a phase shift in a range from 80 to 100 degrees, for example a phase shift in a range from 85 to 95 degrees.

In some embodiments, the processor 102 may be configured to detect tissue inflammation if the determined amplitude of at least one of the plurality of PPG signals is greater than a threshold amplitude and the detected phase shift is greater than a threshold phase shift. In some embodiments, this detection can be achieved using a logic gate (e.g. the "AND" logic gate) or a more complex algorithm (e.g. optimal weighting factors may be learned from a collected data set about tissue inflammation detection).

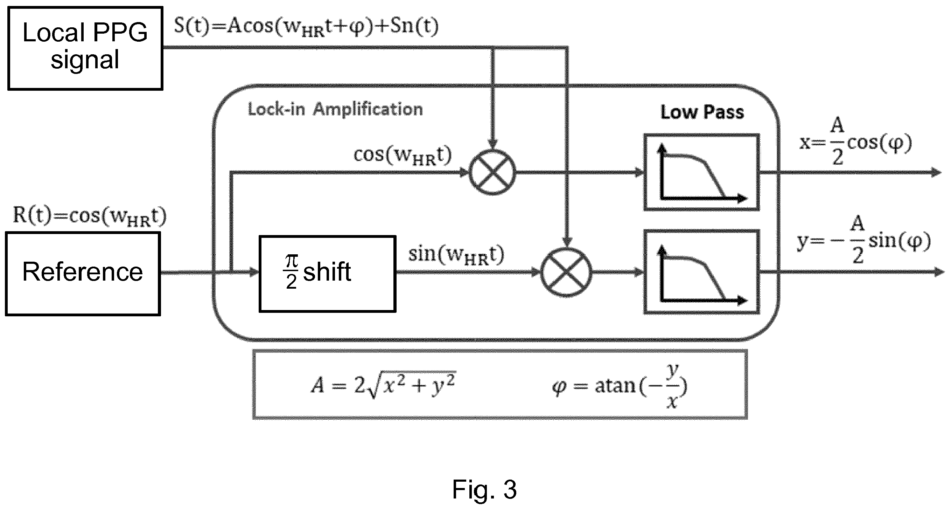

Fig. 3 illustrates an example method for determining the amplitude and phase shift of each of the plurality of PPG signals. The example method uses lock-in amplification. As the plurality of PPG signals are indicative of light detected in a region of tissue at a plurality of respective locations within the region, the plurality of PPG signals can be referred to as a plurality of "local" PPG signals. As illustrated in Fig. 3, the input to the method is a reference signal R(t) and a local PPG signal S(t). The reference signal R(t) can be expressed as:

$$R(t) = \cos(w_{HR}t),$$

where $w_{HR}$ is a heart rate frequency and $t$ is time. The reference signal R(t) can be modulated at the heart rate frequency $w_{HR}$ and obtained by averaging all the local PPG signals.

The local PPG signal S(t) can be expressed as:

$$S(t) = A\cos(w_{HR}t + \varphi) + Sn(t),$$

where $A$ is the amplitude of the modulating heart rate frequency $w_{HR}$, $\varphi$ is the phase shift of the local PPG signal S(t) with respect to the reference signal R(t), Sn(t) contains all the other frequency components, and $t$ is time. R(t) and S(t) are fed into the lock-in amplification block and S(t) is multiplied by R(t), namely by $\cos(w_{HR}t)$, and R(t) is phase shifted by 90 degrees, namely to $\sin(w_{HR}t)$. The two output signals of these operations are then low-pass filtered. The low-pass filtering may, for example, be performed by averaging the two output signals in time, but it will be understood that any other form of low-pass filtering may instead be used.

As a result of the low pass filtering, two values $x$ and $y$ are obtained as $x = \dfrac{A}{2}\cos(\varphi)$, $y = -\dfrac{A}{2}\sin(\varphi)$ respectively. These values are correlated to the amplitude $A$ of the local PPG signal and to the phase shift $\varphi$ of the local PPG signal with respect to R(t). By combining the values x and y, the amplitude $A$ of the local PPG signal and the phase shift $\varphi$ of the local PPG signal can be expressed as follows:

$$A = 2\sqrt{x^2 + y^2} \text{ and } \varphi = \operatorname{atan}\left(-\frac{x}{y}\right) \text{ respectively.}$$

Thus, for each of the plurality of PPG signals a value that represents the amplitude of the PPG signal and a value that represents the phase shift of the PPG signal can be determined.

Although not illustrated in Fig. 2, in some embodiments where the amplitude of each of the plurality of PPG signals is determined, the processor 102 may be configured to generate an amplitude map representative of the determined amplitude of each of the plurality of PPG signals. Alternatively or in addition, in some embodiments where the amplitude of each of the plurality of PPG signals is determined, the processor 102 may be configured to generate an amplitude map representative of the determined amplitude of each of the plurality of PPG signals for which the determined amplitude is greater than a threshold amplitude. In this way, a tissue inflammation can be discriminated from background noise in the generated amplitude map. As the plurality of PPG signals are indicative of light detected in the region of tissue at the plurality of respective locations within the region, the amplitude map representative of the determined amplitude of each of the plurality of PPG signals is a spatial map. The amplitude map can provide an indication of how well the tissue perfused spatially. In some embodiments, the amplitude map may be generated by, for each of the plurality of PPG signals, correlating a color with the determined amplitude of the PPG signal and associating that color to the respective location within the region of tissue. A person skilled in the art will be aware of various techniques that can be used to generate an amplitude map.

Alternatively or in addition, although also not illustrated in Fig. 2, in some embodiments where the phase of each of the plurality of PPG signals is determined, the processor 102 may be configured to generate a phase map representative of the determined phase of each of the plurality of PPG signals. Alternatively or in addition, in some embodiments where the phase of each of the plurality of PPG signals is determined, the processor 102 may be configured to generate a phase map representative of the determined phase of each of the plurality of PPG signals for which a phase shift detected in the determined phase is greater than a threshold phase shift. In this way, a tissue inflammation can be discriminated from background noise in the generated phase map. As the plurality of PPG signals are indicative of light detected in the region of tissue at the plurality of respective locations within the region, the phase map representative of the determined phase of each of the plurality of PPG signals is a spatial map. The phase map can provide an indication of the shift in phase of the local PPG signals. In some embodiments, the phase map may be generated by, for each of the plurality of PPG signals, correlating a color with the determined phase of the PPG signal and associating that color to the respective location within the region of tissue. A person skilled in the art will be aware of various techniques that can be used to generate a phase map.

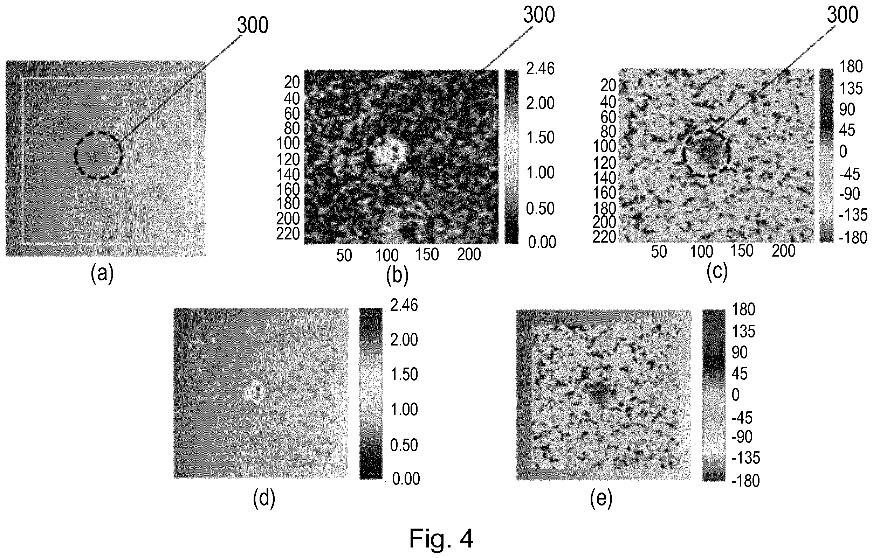

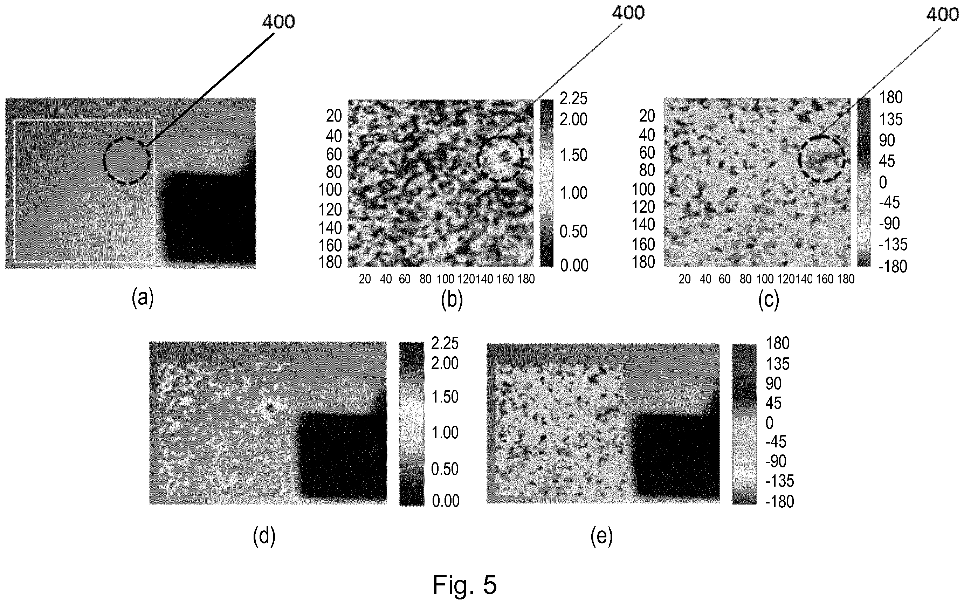

Figs. 4 and 5 are illustrations of amplitude and phase maps according to an embodiment. In more detail, Figs. 4(a) and 5(a) each illustrate an image of a region of tissue, Figs. 4(b) and 5(b) each illustrate a generated amplitude map representative of the determined amplitude of each of the plurality of PPG signals, and Figs. 4(c) and 5(c) each illustrate a generated phase map representative of the determined phase of each of the plurality of PPG signals. As described earlier, the plurality of PPG signals are indicative of light detected in the region of tissue at a plurality of respective locations within the region. A tissue inflammation 300, 400 within the region of tissue is circled in Figs. 4 (a), (b) and (c) and Figs. 5 (a), (b) and (c). In the illustrated examples, the tissue inflammation 300, 400 is a pimple and the tissue is the skin. Figs. 4(d) and 5(d) each illustrate the generated amplitude map overlaid on the image of the region of tissue and Figs. 4(e) and 5(e) each illustrate the generated phase map overlaid on the image of the region of tissue.

As illustrated in Figs. 4 and 5, both the generated amplitude map and the generated phase map are affected by the pimple 300, 400. For example, as shown in Fig. 4(a), the pimple (with pus in the central area) is visible in the circled area of the image of the region of tissue. As can be seen in the amplitude map of Fig. 4(b), there is an increase in the amplitude of the perfusion in the pimple area. Also, as can be seen in the phase map of Fig. 4(c), there is a phase shift in the pimple area. Furthermore, there is an increase in the amplitude of the perfusion in tissue that surrounds the area of pus compared to the area of the pus itself (since the pus is not perfused by blood). On the other hand, Fig. 5 shows an example of detecting the pimple in the early stage. In this case, although the pimple is hardly visible in the circled area of the image of the region of tissue of Fig. 5(a), the local enhanced perfusion is clearly visible in the circled area 400 of the amplitude map of Fig. 5(b) and the circled area 400 of the phase map of Fig. 5(c). Therefore, as illustrated in Figs. 4 and 5, an increase in the amplitude of the PPG signals and phase shift in the PPG signals can provide a good indicator for tissue inflammation (e.g. a pimple) even in the early stages.

In some embodiments where the processor 102 is configured to generate both an amplitude map and a phase map, the processor 102 may also be configured to compare the generated amplitude map and the generated phase map. In some of these embodiments, the processor 102 can be configured to determine a location of the detected tissue inflammation in the region of tissue based on the comparison. For example, in some embodiments, if an amplitude at a location in the generated amplitude map is greater than the threshold amplitude and a phase shift at a corresponding location in the generated phase map is greater than the threshold phase shift, then tissue inflammation can be detected at that location. In some embodiments, this can be achieved using a logic gate (e.g. the "AND" logic gate) or a more complex algorithm (e.g. optimal weighting factors may be learned from a collected data set about tissue inflammation detection).

In some of these embodiments, the processor 102 may also be configured to generate a tissue inflammation map representative of the determined location of the detected tissue inflammation in the region of tissue.

In some embodiments, the processor 102 may be configured to acquire an image (e.g. an RGB image) of the region of tissue and indicate the determined location of the detected tissue inflammation from the generated tissue inflammation map on the acquired image of the region of tissue. For example, in some embodiments, the processor 102 may be configured to indicate the determined location of the detected tissue inflammation on the acquired image of the region of tissue by being configured to overlay the generated tissue inflammation map on the acquired image of the region of tissue.

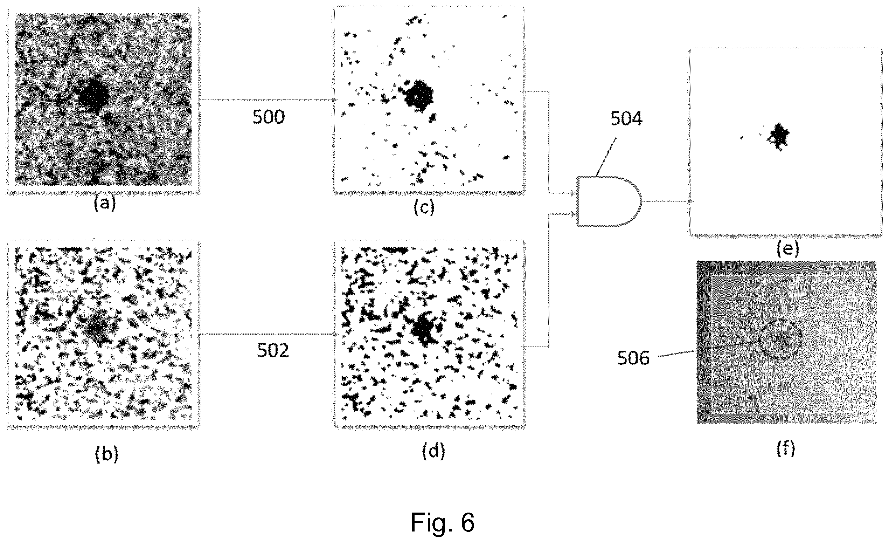

Fig. 6 is an illustration of amplitude and phase maps according to an embodiment where amplitude and phase maps are compared (e.g. in the manner described earlier) and a location of a detected tissue inflammation in a region of tissue is determined. In more detail, Fig. 6(a) illustrates a generated amplitude map representative of the determined amplitude of each of the plurality of PPG signals and Fig. 6(b) illustrates a generated phase map representative of the determined phase of each of the plurality of PPG signals. As described earlier, the plurality of PPG signals are indicative of light detected in the region of tissue at a plurality of respective locations within the region.

At arrow 500 of Fig. 6, the threshold amplitude described earlier is applied to the generated amplitude map of Fig. 6(a). In this way, the tissue inflammation can be discriminated from background noise in the generated amplitude map. Fig. 6(c) thus illustrates a generated amplitude map representative of the determined amplitude of each of the plurality of PPG signals for which the determined amplitude is greater than the threshold amplitude. Similarly, at arrow 502 of Fig. 6, the threshold phase shift described earlier is applied to the generated phase map of Fig. 6(b). In this way, the tissue inflammation can be discriminated from background noise in the generated phase map. Fig. 6(d) thus illustrates a generated phase map representative of the determined phase of each of the plurality of PPG signals for which a phase shift detected in the determined phase is greater than the threshold phase shift.

At block 504 of Fig. 6, the generated amplitude map illustrated in Fig. 6(c) and the generated phase map illustrated in Fig. 6(d) are compared, a location of the detected tissue inflammation in the region of tissue is determined based on the comparison, and a tissue inflammation map representative of the determined location of the detected tissue inflammation in the region of tissue is generated. As described earlier, in some embodiments, if an amplitude at a location in the generated amplitude map of Fig. 6(c) is greater than the threshold amplitude and a phase shift at a corresponding location in the generated phase map Fig. 6(d) is greater than the threshold phase shift, then tissue inflammation can be detected at that location (which is then represented at the corresponding location in the tissue inflammation map). In some embodiments, this can be achieved using a logic gate (e.g. the "AND" logic gate) as illustrated by the shape of block 504 in Fig. 6 or a more complex algorithm (e.g. optimal weighting factors may be learned from a collected data set about tissue inflammation detection). In effect, the generated amplitude map illustrated in Fig. 6(c) and the generated phase map illustrated in Fig. 6(d) are combined.

Fig. 6(e) illustrates the generated tissue inflammation map. As can be seen from Fig. 6(e), the tissue inflammation is clearly visible in the generated tissue inflammation map and the background noise is not visible. In this way, tissue inflammation can be more accurately detected. Fig. 6(f) illustrates the generated tissue inflammation map overlaid on (or overlapping) an acquired image of the region of tissue. It can thus be seen that tissue inflammation (e.g. a pimple) is detected.

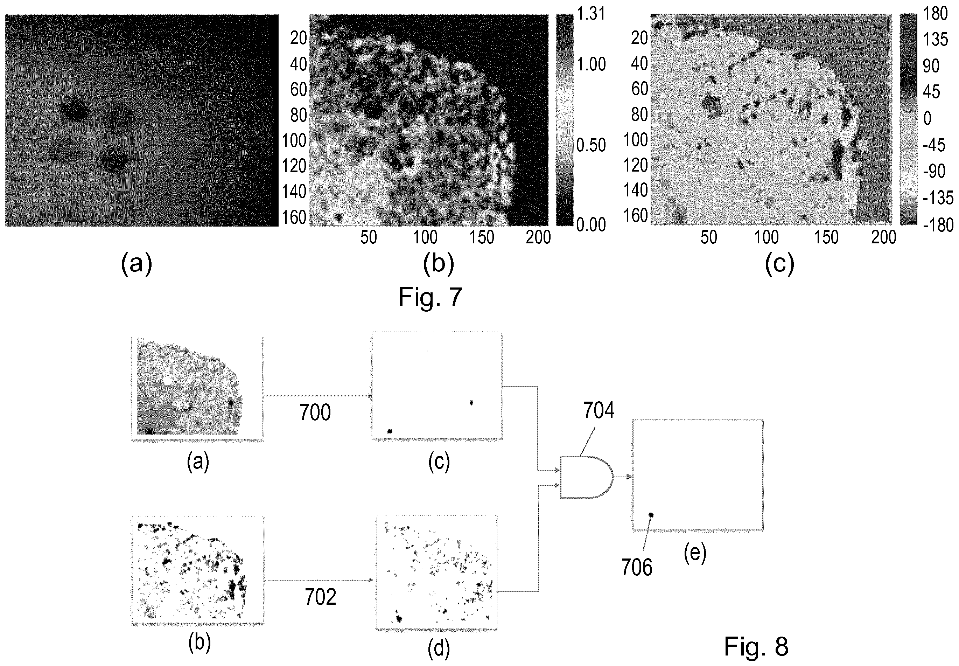

Fig. 7 is an illustration of amplitude and phase maps according to an embodiment. In more detail, Fig. 7(a) illustrates an image of a region of tissue. In this example, the tissue is skin on a forehead of a subject. Four colored dots are drawn in ink on the forehead. Fig. 7(b) illustrates a generated amplitude map representative of the determined amplitude of each of the plurality of PPG signals and Fig. 7(c) illustrates a generated phase map representative of the determined phase of each of the plurality of PPG signals. The plurality of PPG signals are indicative of light detected in the region on the forehead of the subject of skin at a plurality of respective locations within the region. A black dot is seen as background by the processor 102 of the apparatus 100, which occludes the skin and thus no light (and thus no perfusion) is detected in the amplitude map of Fig. 7(b) at the location of the black dot. The phase in the area of the black dot in the phase map of Fig. 7(c) is also the same as the background. The processor 102 of the apparatus 100 detects less perfusion on the areas of the dots (since the ink covers the skin). The phase map of Fig. 7(c) shows no change in perfusion on the areas of the dots. On the borders of the dots, a variation in perfusion is detected but only due to an abrupt change in color between the skin and ink. By examining both the amplitude of and phase shift in the PPG signals, any non-inflammatory tissue (e.g. tattoos, make-up, spider veins, or other non-inflammatory tissue) is not misclassified as inflammatory tissue (e.g. pimples, or other inflammatory tissue).

Fig. 8 is an illustration of amplitude and phase maps according to an embodiment where amplitude and phase maps are compared (e.g. in the manner described earlier) and a location of a detected tissue inflammation in a region of tissue determined. In this example, the region of tissue is the region of skin on the forehead of the subject on which the four colored dots are drawn in ink as illustrated in Fig. 7. In more detail, Fig. 8(a) illustrates a generated amplitude map representative of the determined amplitude of each of the plurality of PPG signals and Fig. 8(b) illustrates a generated phase map representative of the determined phase of each of the plurality of PPG signals. The plurality of PPG signals are indicative of light detected in the region of skin on the forehead at a plurality of respective locations within the region.

At arrow 700 of Fig. 8, the threshold amplitude described earlier is applied to the generated amplitude map of Fig. 8(a). In this way, the tissue inflammation can be discriminated from background noise in the generated amplitude map. Fig. 8(c) thus illustrates a generated amplitude map representative of the determined amplitude of each of the plurality of PPG signals for which the determined amplitude is greater than the threshold amplitude. Similarly, at arrow 702 of Fig. 8, the threshold phase shift described earlier is applied to the generated phase map of Fig. 8(b). In this way, the tissue inflammation can be discriminated from background noise in the generated phase map. Fig. 8(d) thus illustrates a generated phase map representative of the determined phase of each of each of the plurality of PPG signals for which a phase shift detected in the determined phase is greater than the threshold phase shift.

At block 704 of Fig. 8, the generated amplitude map illustrated in Fig. 8(c) and the generated phase map illustrated in Fig. 8(d) are compared, a location of detected tissue inflammation in the region of skin on the forehead is determined based on the comparison, and a tissue inflammation map representative of the determined location of the detected tissue inflammation in the region of skin on the forehead is generated. As described earlier, in some embodiments, if an amplitude at a location in the generated amplitude map of Fig. 8(c) is greater than the threshold amplitude and a phase shift at a corresponding location in the generated phase map Fig. 8(d) is greater than the threshold phase shift, then tissue inflammation can be detected at that location (which is then represented at the corresponding location in the tissue inflammation map). In some embodiments, this can be achieved using a logic gate (e.g. the "AND" logic gate) as illustrated by the shape of block 704 in Fig. 8 or a more complex algorithm (e.g. optimal weighting factors may be learned from a collected data set about tissue inflammation detection). In effect, the generated amplitude map illustrated in Fig. 8(c) and the generated phase map illustrated in Fig. 8(d) are combined. Fig. 8(e) illustrates the generated tissue inflammation map. As can be seen from Fig. 8(e), the tissue inflammation 706 (which, in this example, is a potential pimple in the early stages) is clearly visible in the generated tissue inflammation map and the background noise (including the colored dots) is not visible. Thus, as illustrated by Fig. 8, tissue inflammation can be more accurately detected.

There is also described a computer program product comprising a computer readable medium. The computer readable medium has computer readable code embodied therein. The computer readable code is configured such that, on execution by a suitable computer or processor, the computer or processor is caused to perform the method described herein. The computer readable medium may be, for example, any entity or device capable of carrying the computer program product. For example, the computer readable medium may include a data storage, such as a ROM (such as a CD-ROM or a semiconductor ROM) or a magnetic recording medium (such as a hard disk). Furthermore, the computer readable medium may be a transmissible carrier, such as an electric or optical signal, which may be conveyed via electric or optical cable or by radio or other means. When the computer program product is embodied in such a signal, the computer readable medium may be constituted by such a cable or other device or means. Alternatively, the computer readable medium may be an integrated circuit in which the computer program product is embedded, the integrated circuit being adapted to perform, or used in the performance of, the method described herein.

Therefore, there is described herein an apparatus 100, a method 200 and a computer program product that address the limitations associated with the existing techniques. An improved apparatus and method for detecting tissue inflammation is thus described. The detection of tissue inflammation in the manner described herein can enable better detection of infections, wound healing, skin irritation, and other conditions involving tissue inflammation.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. An apparatus (100) for detecting tissue inflammation, the apparatus (100) comprising a processor (102) configured to:

   acquire, from at least one sensor (104), a plurality of photoplethysmography, PPG, signals indicative of light detected in a region of tissue at a plurality of respective locations within the region;
process the acquired plurality of PPG signals to determine an amplitude and a phase of each of the plurality of PPG signals; and
detect tissue inflammation (300, 400) based on the determined amplitude and phase of each of the plurality of PPG signals.

2. The apparatus (100) as claimed in claim 1, wherein the processor (102) is configured to:
detect tissue inflammation (300, 400) if the determined amplitude of at least one of the plurality of PPG signals is greater than a threshold amplitude.

3. The apparatus (100) as claimed in any of the preceding claims, wherein the amplitude of each of the plurality of PPG signals is the amplitude of the PPG signal normalized by a direct current component of the PPG signal.

4. The apparatus (100) as claimed in any of the preceding claims, wherein the processor (102) is configured to:
detect tissue inflammation (300, 400) if a phase shift is detected in the determined phase of at least one of the plurality of PPG signals.

5. The apparatus (100) as claimed in claim 4, wherein the processor (102) is configured to:
detect tissue inflammation (300, 400) if the detected phase shift is greater than a threshold phase shift.

6. The apparatus (100) as claimed in claim 5, wherein the threshold phase shift is a phase shift in a range from 45 degrees to 135 degrees.

7. The apparatus (100) as claimed in any of the preceding claims, wherein the processor (102) is configured to:
generate an amplitude map representative of the determined amplitude of each of the plurality of PPG signals.

8. The apparatus (100) as claimed in any of the preceding claims, wherein the processor (102) is configured to:
generate an amplitude map representative of the determined amplitude of each of the plurality of PPG signals for which the determined amplitude is greater than a threshold amplitude.

9. The apparatus (100) as claimed in any of the preceding claims, wherein the processor (102) is configured to:
generate a phase map representative of the determined phase of each of the plurality of PPG signals.

10. The apparatus (100) as claimed in any of the preceding claims, wherein the processor (102) is configured to:
generate a phase map representative of the determined phase of each of the plurality of PPG signals for which a phase shift detected in the determined phase is greater than a threshold phase shift.

11. The apparatus (100) as claimed in claim 9 when dependent on claim 7 or as claimed in claim 10 when dependent on claim 8, wherein the processor (102) is configured to:

compare the generated amplitude map and the generated phase map; and
determine a location of the detected tissue inflammation (300, 400) in the region of tissue based on the comparison.

12. The apparatus (100) as claimed in claim 11, wherein the processor (102) is configured to:
generate a tissue inflammation map representative of the determined location of the detected tissue inflammation (300, 400) in the region of tissue.

13. The apparatus (100) as claimed in claim 12, wherein the processor (102) is configured to:

acquire an image of the region of tissue; and
indicate the determined location of the detected tissue inflammation (300, 400) from the generated tissue inflammation map on the acquired image of the region of tissue.

14. A method (200) for detecting tissue inflammation, the method (200) comprising:

acquiring (202) a plurality of PPG signals indicative of light detected in a region of tissue at a plurality of respective locations within the region;
processing (204) the acquired plurality of PPG signals to determine an amplitude and a phase of each of the plurality of PPG signals; and
detecting (206) tissue inflammation (300, 400) based on the determined amplitude and phase of each of the plurality of PPG signals.

15. A computer program product comprising a computer readable medium, the computer readable medium having computer readable code embodied therein, the computer readable code being configured such that, on execution by a suitable computer or processor, the computer or processor is caused to perform the method as claimed in claim 14.

Fig. 1

200

202

204

206

Fig. 2

Fig. 3

EP 3 666 176 A1

Fig. 4

Fig. 5

15

Fig. 6

Fig. 7

Fig. 8

Europäisches
Patentamt

European
Patent Office

Office européen
des brevets

**EUROPEAN SEARCH REPORT**

Application Number

EP 18 21 2799

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 384 855 A1 (KONINKLIJKE PHILIPS NV [NL]) 10 October 2018 (2018-10-10) | 1,4,6,7, 9,14,15 | INV. A61B5/00 |
| Y | * figure 1 *<br>* paragraphs [0034], [0038], [0049] * | 2,3,5,8, 10-13 | |
| Y | WO 2016/096591 A1 (KONINKL PHILIPS NV [NL]) 23 June 2016 (2016-06-23)<br>* figure 6b *<br>* page 2, line 15 - line 16 *<br>* page 14, line 26 - line 30 *<br>* page 15, line 30 - line 32 * | 2,5,8, 10-13 | |
| Y | HSUAN SHEN ET AL: "A novel 500 samples/sec 4 by 4 photoplethysmography (PPG) array based cosmetic chip design", COMPUTER SYMPOSIUM (ICS), 2010 INTERNATIONAL, IEEE, PISCATAWAY, NJ, USA, 16 December 2010 (2010-12-16), pages 942-947, XP031847637, ISBN: 978-1-4244-7639-8<br>* figures 14-16 *<br>* page 944, line 1 - line 8 *<br>* page 947, line 7 - line 13 * | 3 | |
| A | ENRICO M. STADERINI ET AL: "Near Infrared Device for Tissue Inflammation Evaluation", MATERIALS SCIENCE FORUM, vol. 879, 1 November 2016 (2016-11-01), pages 2361-2364, XP055592717, DOI: 10.4028/www.scientific.net/MSF.879.2361<br>* figures 2, 6, 7 * | 1,3, 11-13 | TECHNICAL FIELDS SEARCHED (IPC)<br>A61B |

The present search report has been drawn up for all claims

| Place of search | Date of completion of the search | Examiner |
|---|---|---|
| The Hague | 29 May 2019 | Brendemühl, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 3 666 176 A1

## ANNEX TO THE EUROPEAN SEARCH REPORT
## ON EUROPEAN PATENT APPLICATION NO.

EP 18 21 2799

29-05-2019

| Patent document cited in search report | | Publication date | Patent family member(s) | | Publication date |
|---|---|---|---|---|---|
| EP 3384855 | A1 | 10-10-2018 | NONE | | |
| WO 2016096591 | A1 | 23-06-2016 | CN | 106999057 A | 01-08-2017 |
| | | | EP | 3232910 A1 | 25-10-2017 |
| | | | JP | 2018504174 A | 15-02-2018 |
| | | | US | 2017347938 A1 | 07-12-2017 |
| | | | WO | 2016096591 A1 | 23-06-2016 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007103795 A